# EUROPEAN PATENT SPECIFICATION

(11) **EP 3 131 470 B1**
(45) Date of publication and mention of the grant of the patent: **07.08.2019**
(21) Application number: 15779605.3
(22) Date of filing: 20.03.2015
(51) Int. Cl.: A61B 8/00, A61B 8/08, A61B 8/12

(54) **ULTRASONIC IMAGING APPARATUS**
ULTRASCHALLABBILDUNGSVORRICHTUNG
APPAREIL D'IMAGERIE ULTRASONORE

(30) Priority: 15.04.2014 KR 20140044685
(43) Date of publication of application: 22.02.2017
(73) Proprietor: Samsung Electronics Co., Ltd., Suwon-si, Gyeonggi-do 443-742 (KR)
(72) Inventor: CHO, Kyung Il, Seoul 138-788 (KR); KIM, Bae Hyung, Yongin-si Gyeonggi-do 446-760 (KR); KIM, Young Il, Suwon-si Gyeonggi-do 440-723 (KR); SONG, Jong Keun, Yongin-si Gyeonggi-do 446-882 (KR); LEE, Seung Heun, Seongnam-si Gyeonggi-do 463-808 (KR)
(74) Representative: Gulde & Partner
(86) International application number: PCT/KR2015/002743
(87) International publication number: WO 2015/160100

(56) References cited:
- WO-A2-2013/140311
- US-A- 5 555 887
- US-A1- 2006 173 344
- US-A1- 2008 300 492
- US-A1- 2008 306 389
- US-A1- 2009 030 325
- US-A1- 2009 088 646
- US-A1- 2013 286 593

## Description

### [Technical Field]

Exemplary embodiments relate to an ultrasonic imaging apparatus, and more particularly to an ultrasonic imaging apparatus capable of efficiently dissipating heat from an ultrasonic probe during diagnosis.

### [Background Art]

Medical ultrasonic imaging apparatuses are generally classified into non-invasive type ultrasonic imaging apparatuses and invasive type ultrasonic imaging apparatuses. One of the representative invasive type ultrasonic imaging apparatuses is a transesophageal echocardiography (TEE) imaging apparatus.

Transesophageal echocardiography is an established technique in the area of cardiac imaging, and involves insertion of a probe into a subject's esophagus. The probe, which is affixed to a distal end portion of a long tube, is positioned near a heart through a subject's esophagus, irradiates ultrasonic waves toward a heart and surrounding tissues (i.e., an object to be diagnosed), and obtains images of heart chambers, valves and surrounding tissues by receiving an ultrasonic echo signal which is reflected from the object.

The tube used in the transesophageal echocardiography may have proper rigidity and flexibility to be inserted into a subject (e.g., human body) via a subject's esophagus. Further, there is provided a bending part which is bendable between the tube and the probe so that the probe may pass through a curved esophagus and may be disposed at the optimum position for heart diagnosis.

US 2008/306389 A1 relates to an ultrasonic endoscope to be used for body cavity examination of upper digestive organs, bronchial tube, and so on, and the present invention further relates to an ultrasonic endoscopic apparatus including the ultrasonic endoscope.

US 2009/030325 A1 relates to an ultrasonic probe to be used when intracavitary scanning or extracavitary scanning is performed on an object to be inspected, and an ultrasonic endoscope to be inserted into a body cavity of the object.

### [Disclosure]

### [Technical Problem]

It is an aspect of one or more exemplary embodiments to provide an ultrasonic imaging apparatus capable of effectively dissipating heat from a probe during diagnosis.

Additional aspects of the exemplary embodiments will be set forth in part in the description which follows and, in part, will be obvious from the description, or may be learned by practice of the exemplary embodiments.

### [Technical Solution]

In accordance with one aspect of one or more exemplary embodiments, an ultrasonic imaging apparatus comprises an ultrasonic probe arranged at a distal end portion of the ultrasonic imaging apparatus a bending part connected to the ultrasonic probe and configured to be bendable, and a heat transferer connected to the heat spreader and configured to transfer heat absorbed by the heat spreader toward the bending part,. Heat generated from the ultrasonic probe is dissipated toward the bending part. The ultrasonic probe includes: an ultrasonic transducer, an integrated circuit which includes a front surface and a rear surface, the ultrasonic transducer being arranged on the front surface of the integrated circuit, a printed circuit board which includes a front surface and a rear surface, the rear surface of the integrated circuit being arranged on the front surface of the printed circuit board, and
a heat spreader configured to absorb heat generated by the integrated circuit, the heat spreader being arranged on the rear surface of the printed circuit board. The heat transferer includes a refrigerant circulation device, wherein the refrigerant circulation device includes a refrigerant circulation tube configured to circulate a refrigerant therethrough. The heat spreader includes: an inner casing coupled to the refrigerant circulation tube and having a passage (P) formed therein configured to circulate the refrigerant therethrough; and an outer casing formed with a coupling recess (C) and having a shape which corresponds to the inner casing.

### [Advantageous Effects]

As is apparent from the above description, the thermal stability of the ultrasonic probe may be enhanced by efficiently dissipating heat generated from the ultrasonic probe by using a capacitive micromachined ultrasonic transducer (cMUT).

Further, because it is not necessary to form a backing layer for improvement of residual vibration characteristics, the structure and manufacturing processes may be simplified.

### [Description of Drawings]

These and/or other aspects of the exemplary embodiments will become apparent and more readily appreciated from the following description of the exemplary embodiments, taken in conjunction with the accompanying drawings of which:
FIG. 1 is a view illustrating a constitution of an ultrasonic imaging apparatus, according to an exemplary embodiment;
FIG. 2 is a cross-sectional view illustrating a structure of an ultrasonic probe, according to an exemplary embodiment;
FIG. 3 is a plan view illustrating a structure of an ultrasonic probe, according to an exemplary embodiment;
FIG. 4 is a cross-sectional view illustrating a structure of an ultrasonic probe, according to another exemplary embodiment;
FIG. 5 is a plan view illustrating a structure of an ultrasonic probe, according to another exemplary embodiment;
FIG. 6 is a conceptual view illustrating a constitution of an ultrasonic transducer, according to an exemplary embodiment;
FIG. 7 is a cross-sectional view illustrating a structure of an ultrasonic probe in which an opening is formed at a printed circuit board;
FIG. 8 is a cross-sectional view illustrating a structure of an ultrasonic probe in which a plurality of vias are provided at a printed circuit board;
FIG. 9 is a view illustrating a case in which graphite is used as a heat transfer;
FIG. 10 is a view illustrating a case in which a refrigerant circulation device is used as a heat transfer;
FIG. 11 is a conceptual view illustrating a refrigerant circulating through an internal passage of a heat spreader; and
FIG. 12 is a perspective view illustrating a structure of a heat spreader having an inner casing and an outer casing.

### [Best Mode]

Now, exemplary embodiments will be described in detail with reference to the the annexed drawings. In the drawings, the same or similar elements are denoted by the same reference numerals even though they are depicted in different drawings. In the following description, a detailed description of known functions and configurations incorporated herein will be omitted when it may make the subject matter of the exemplary embodiments rather unclear. It will be understood that the terms first, second, etc. may be used herein to distinguish one component from another component and these components should not be limited by these terms.

Hereinafter, the exemplary embodiments will be described based on a transesophageal echocardiography (TEE) apparatus, however, the exemplary embodiments are not limited to the transesophageal echocardiography (TEE) apparatus, but may be applied to all kinds of ultrasonic imaging apparatuses.

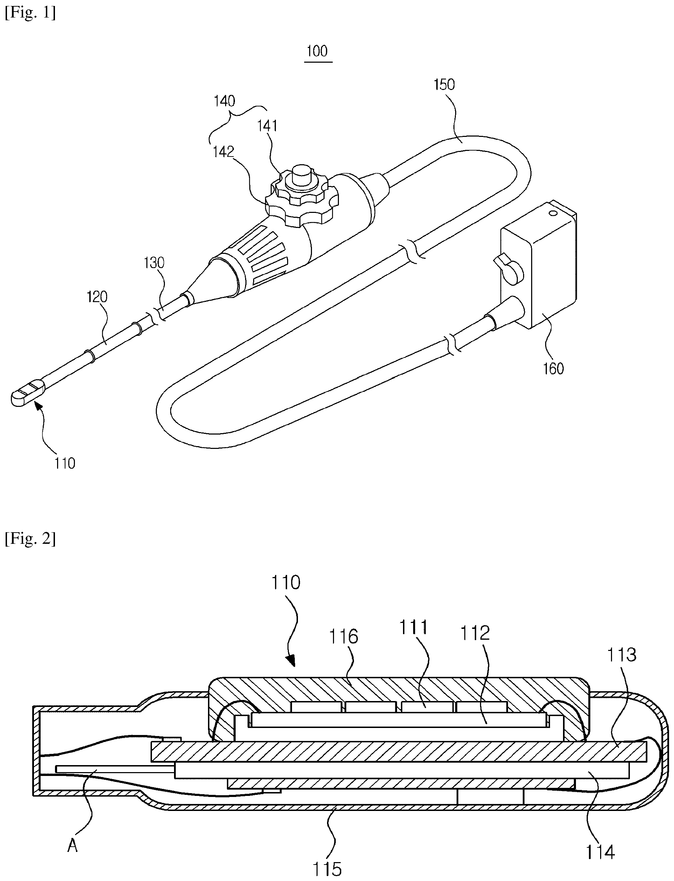

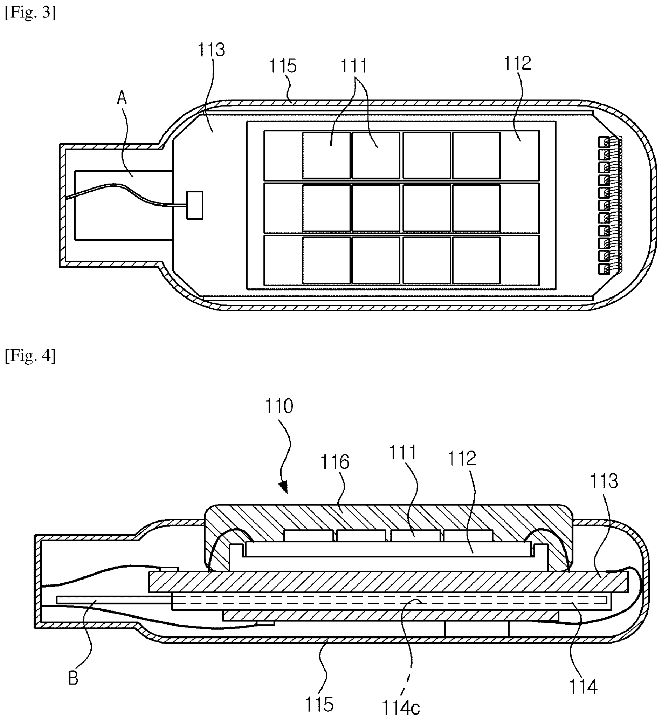

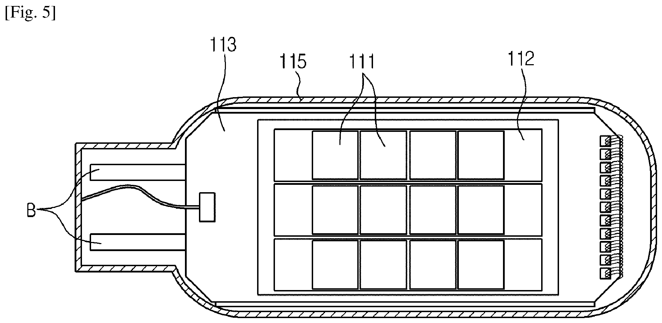

FIG. 1 is a view illustrating a constitution of an ultrasonic imaging apparatus according to an exemplary embodiment, FIG. 2 is a cross-sectional view illustrating a structure of an ultrasonic probe according to an exemplary embodiment, and FIG. 3 is a plan view illustrating a structure of an ultrasonic probe according to an exemplary embodiment. FIG. 4 is a cross-sectional view illustrating a structure of an ultrasonic probe according to another exemplary embodiment, and FIG. 5 is a plan view illustrating a structure of an ultrasonic probe according to another exemplary embodiment.

Referring to FIG. 1, an ultrasonic imaging apparatus 100 according to an exemplary embodiment may comprise an ultrasonic probe 110, an insertion tube 130 and a manipulation device 140.

The ultrasonic probe 110 is configured to irradiate ultrasonic waves toward an object and to acquire an image of the interior of the object by receiving an ultrasonic echo signal which is reflected from the object. As shown in FIG. 2, the ultrasonic probe 110 may include an ultrasonic transducer 111, an integrated circuit 112, a printed circuit board 113, and a heat spreader 114.

The ultrasonic transducer 111 functions to convert an electrical signal supplied from the outside into a mechanical vibration energy in order to generate ultrasonic waves, and also functions to convert a vibration transmitted from the outside into an electrical signal.

In this exemplary embodiment, as the ultrasonic transducer 111, a capacitive micromachined ultrasonic transducer (cMUT) may be employed, however, the exemplary embodiments are not limited thereto. As the ultrasonic transducer 111, a piezo-electronic transducer (PZT) may be employed. Hereinafter, for convenience of explanation, the exemplary embodiments will be described with reference to a case in which a cMUT is used as the ultrasonic transducer 111.

The cMUT is a relatively new concept in the field of ultrasonic transducers which transmit and receive ultrasonic waves by using vibrations of hundreds or thousands of microprocessed thin films. Such a cMUT may be manufactured based on micro electro mechanical system (MEMS) technology. A capacitor is formed by forming a lower electrode and an insulating layer on a semiconductor substrate commonly used in semiconductor manufacturing processes, forming an air gap on the insulating layer including the lower electrode, forming a thin film with a thickness of several to thousands of angstroms on the air gap, and forming an upper electrode on the thin film.

When an alternating current is applied to the capacitor, ultrasonic waves are generated by vibration of the thin film. Conversely, when the thin film is caused to vibrate by external ultrasonic waves, the capacitance of the capacitor varies. By detecting such capacitance variation, ultrasonic waves are detected.

Because each of the thin films of the cMUT has a diameter of dozens of micrometers (µm), an array of tens of thousands of thin films has a size of approximately several millimeters. In addition, because tens of thousands of thin films may be accurately arranged at desired positions via a single semiconductor manufacturing process, and the cMUT is electrically connected to the integrated circuit by chip bonding, such as flip-chip bonding, in order to apply electrical signals to the cMUT, process complexity due to wiring may be overcome.

In addition, because the cMUT has poor vibration transmissibility due to the air gap, it is not necessary to form a backing layer for improvement of residual vibration characteristics on a rear surface of the printed circuit board 113, which will be described below. Accordingly, the structure and manufacturing processes may be simplified.

Further, because the cMUT has broadband characteristics, the cMUT may obtain an image of higher resolution than the resolution of a corresponding image obtained by a piezo-electronic transducer. Because the cMUT is adequate for manufacturing a transducer having a two-dimensional array structure, development of a multichannel transducer may be facilitated, and thus a three-dimensional image may be realized in the field of the ultrasonic imaging apparatuses.

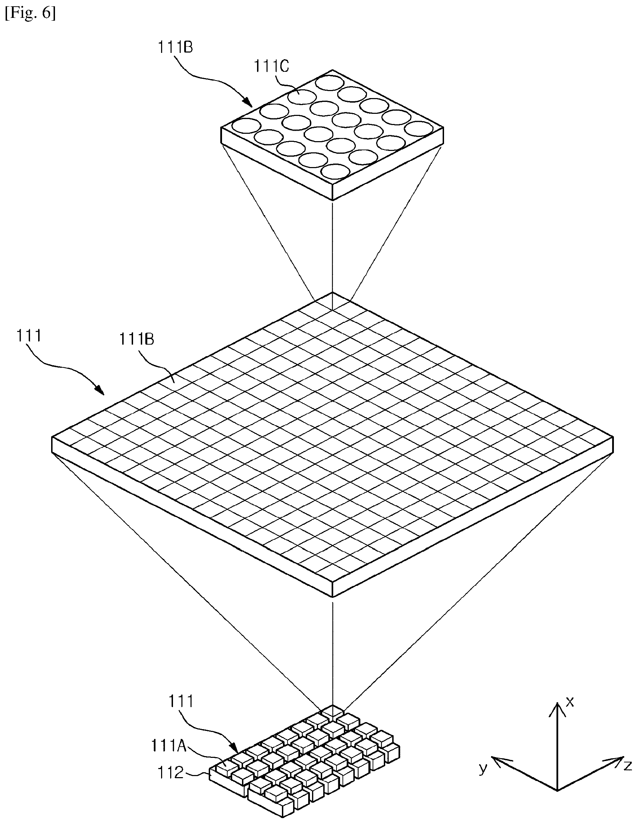

The ultrasonic transducer 111, as which the cMUT is used, may have a two-dimensional array structure, as shown in FIG. 6. In particular, a basic component which constitutes the ultrasonic transducer 111 is referred to as a tile 111A.

The tile 111A includes elements 111B which are arranged in a two-dimensional array. In each element 111B, a plurality of thin films 111C, which vibrate in response to an electrical signal applied thereto, may be arranged in a two-dimensional array.

For example, as shown in FIG. 6, if the ultrasonic transducer 111 has a 4x8 two-dimensional array structure which includes 32 tiles 111A, each of the tiles 111A may have a 16x16 two-dimensional array structure which includes 256 elements 111B, and each of the elements 12 may include 20 thin films 111C which vibrate in response to an electrical signal applied thereto in order to generate ultrasonic waves, and therefore, the ultrasonic transducer 111 includes a total of 163,840 thin films 111C.

As such, the cMUT is adequate for manufacturing the transducer having a two-dimensional array structure, which facilitates development of a multichannel transducer. However, while an amount of heat which is generated in electrical circuits which are designed for driving the ultrasonic probe 110 which includes transducers having a relatively small number of channels is approximately equal to one watt (i.e., 1 W), which may be naturally dissipated through a probe case, an amount of heat which is generated in electrical circuits which are designed for driving the ultrasonic probe 110 which includes multichannel transducers is approximately equal to 7 W. Thus, there is a need to develop techniques for dissipating heat generated by the ultrasonic probe 110 and for cooling the ultrasonic probe 110.

Therefore, one or more exemplary embodiments is designed to effectively dissipate heat generated by the ultrasonic probe 110.

The integrated circuit 112 is configured to drive the ultrasonic transducer 111 by applying an electrical signal to the ultrasonic transducer 111 in order to generate an ultrasonic signal and to detect an electrical signal output from the ultrasonic transducer 111 by the ultrasonic signal transmitted to the ultrasonic transducer 111 from the outside. As shown in FIG. 2, the integrated circuit 112 has a front surface and a rear surface, and the ultrasonic transducer 111 may be arranged on the front surface of the integrated circuit 112.

The ultrasonic transducer 111 may be installed on the front surface of the integrated circuit 111, for example, by flip-chip bonding as described above, however, the exemplary embodiments are not limited thereto.

Although not illustrated, the integrated circuit 112 may include an analog beamformer, an analog-to-digital converter (ADC) and a digital beamformer, however, the exemplary embodiments are not limited thereto.

The analog beamformer functions to output electrical signals to the ultrasonic transducer 111, thereby enabling the thin films 111C constituting the ultrasonic transducer 111 to respectively generate ultrasonic signals. In this aspect, the electrical signals output from the analog beamformer correspond to the respective thin films 111C, and thus the same number of the electrical signals as the number of the thin films may be output.

In order to cause the ultrasonic signals generated by the ultrasonic transducer 111 to overlap at a focusing point, the ultrasonic signals should arrive at the focusing point at the same time. However, there may be a difference in distances between the respective thin films 111C of the ultrasonic transducer 111 and the focusing point, and such a difference in distance may cause a predetermined gap of time in which the ultrasonic signals arrive at the focusing point.

Accordingly, in order to ensure that ultrasonic signals generated by the ultrasonic transducers 111 arrive at the focusing point at approximately the same time, the thin film positioned relatively far from the focusing point generates an ultrasonic signal earlier, and the thin film positioned relatively close to the focusing point generates an ultrasonic signal later. To this end, the analog beamformer acts to output electrical signals to be applied to the respective thin films 111C at different points of time. As a result, the respective thin films 111C constituting the ultrasonic transducer 111 generate ultrasonic signals at different points of time.

When the ultrasonic signals arrive at the focusing point, the ultrasonic signals may be reflected by the focusing point and/or by the surrounding body tissues. When the reflected return signals arrive at the ultrasonic transducer 111, the ultrasonic transducer 111 converts the return signals into electrical signals, and transmits the converted electrical signals to the analog beamformer. In this aspect, the same number of the electrical signals as the number of the thin films 111C constituting the ultrasonic transducer 111 may be generated.

When receiving the electrical signals converted by the ultrasonic transducer 111, the analog beamformer adjusts phases with respect to the respective electrical signals in order to overlap the electrical signals to a single signal. The reason for adjusting the phases by the analog beamformer is that the reflected signals arrive at the respective thin films 111C of the ultrasonic transducer 111 at different points of time. Such a single signal generated as a result of the overlapping is converted into a digital signal through the ADC.

The digital beamformer synthesizes the digital signals converted through the ADC according to the precalculated time delay value for digital beamforming in order to form a receiving beam.

By virtue of the analog beamformer, there is no need for a plurality of sampling devices which respectively correspond to the thin films included in the ultrasonic transducer 111, and thus, an increase in complexity of hardware may be avoided. Further, since the analog beamformer causes the electrical signals converted by the ultrasonic transducer 111 to be focused to a subgroup and to be converted into a digital signal, the number of channels may be reduced, and the diameter of the insertion tube 130 may be decreased.

Application specific integrated circuits (ASIC) may be used as the integrated circuit 112, however, the exemplary embodiments are not limited thereto.

As shown in FIG. 2, the printed circuit board 113 has a front surface and a rear surface, and the rear surface of the integrated circuit 112 may be arranged on the front surface of the printed circuit board 113. The printed circuit board 113 and the integrated circuit 112 may be electrically connected to each other by wire bonding, however, the electrical connection between the printed circuit board 113 and the integrated circuit 112 is not limited to wire bonding.

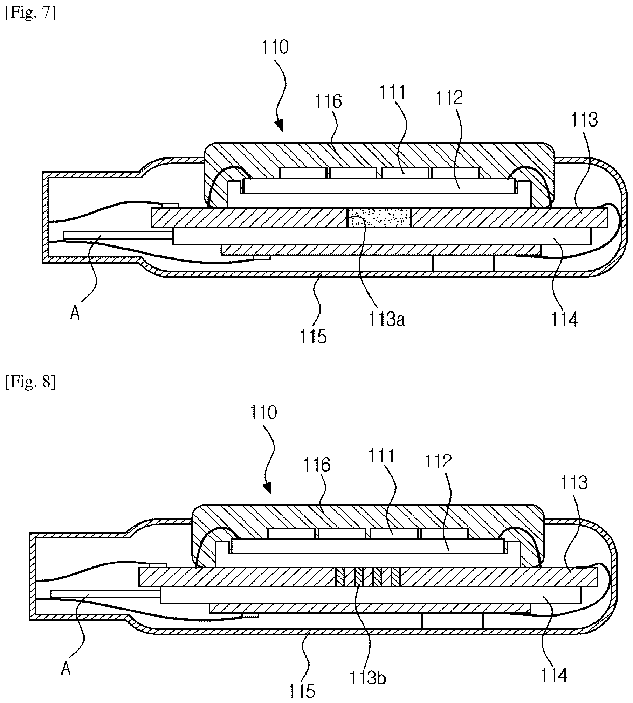

Because the printed circuit board 113 is made of a material having a relatively low thermal conductivity, as shown in FIG. 7, the printed circuit board 113 may be formed with an opening 113a through which a part of the rear surface of the integrated circuit 112 is exposed. A material having a relatively high thermal conductivity, such as, for example, a thermal grease, a phase change material or the like, may be provided in the opening 113a.

As shown in FIG. 8, the printed circuit board 113 may have a plurality of vias 113b which are provided through the printed circuit board 113 in a thickness direction of the printed circuit board 113. The vias 113b may be made of copper having a relatively high thermal conductivity, however, the exemplary embodiments are not limited thereto.

Since the rear surface of the integrated circuit 112 and the heat spreader 114 which will be described below may thermally contact each other through the thermal grease, the phase change material and/or the vias, heat generated by the integrated circuit 112 may be more rapidly absorbed in the heat spreader 114.

The heat spreader 114 is configured to absorb heat generated by the integrated circuit 112 of the ultrasonic probe 110, and may be arranged on the rear surface of the printed circuit board 113.

The heat spreader 114 may be made of a metal, such as, for example, aluminum, however, the exemplary embodiments are not limited thereto. Any material having a relatively high thermal conductivity may be used for the heat spreader 114.

The heat spreader 114 may include a protruding part which extends toward a bending part 120 (refer to FIG. 1). The protruding part functions to dissipate heat generated by the integrated circuit 112 toward the bending part 120. The protruding part may be formed in a plate shape as shown in FIGS. 2 and 3 (see item labeled "A", or may be formed in a pipe shape as shown in FIGS. 4 and 5 (see item labeled "B"), however, the exemplary embodiments are not limited thereto. In the case in which the protruding part is formed in a pipe shape, the protruding part may be provided as a pair as shown in FIG. 5, however, the exemplary embodiments are not limited thereto.

For convenience of explanation, the protruding part formed in a plate shape is denoted by 'A', and the protruding part formed in a pipe shape is denoted by 'B'. The protruding part A or B may be thermally connected to a heat transferer which will be described below.

The ultrasonic probe 110 according to an exemplary embodiment may further include a housing 115 in which the ultrasonic transducer 111, the integrated circuit 112, the printed circuit board 113 and the heat spreader 114 are accommodated. The housing 115 may be made of any one or more of plastic, fiberglass, epoxy, or the like, however, the exemplary embodiments are not limited thereto.

The housing 115 may include a window configured to irradiate ultrasonic signals to the outside and to receive the external ultrasonic signals therethrough. As shown in FIG. 2, the window may be provided with a lens 116. Accordingly, the ultrasonic probe 110 may irradiate ultrasonic signals from the ultrasonic transducer 111 toward an object through the lens 116, and may also receive the ultrasonic signals reflected from the object through the lens 116.

The ultrasonic imaging apparatus 100 according to an exemplary embodiment may further include a heat transferer configured to transfer heat absorbed in the heat spreader 114 from the integrated circuit 112 toward the bending part 120.

As the heat transferer, a refrigerant circulation device or graphite may be employed, however, the exemplary embodiments are not limited thereto.

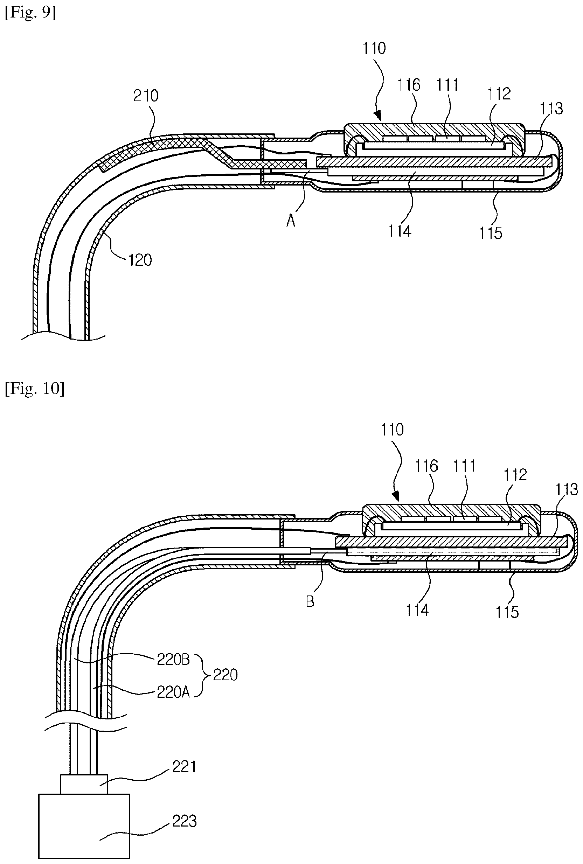

FIG. 9 is a view illustrating a case in which graphite is used as the heat transferer.

Referring to FIG. 9, the protruding part A of the heat spreader 114, more particularly, the plate-shaped protruding part A, and an inner wall of the bending part 120 are connected to each other using graphite 210. In particular, one end portion of the graphite 210 is bonded to the protruding part A of the heat spreader 114, and an opposite end portion of the graphite 210 is bonded to the inner wall of the bending part 120.

The bending part 120 has a multi-joint mechanism which is bendable. As shown in FIG. 1, the bending part 120 may be arranged between the ultrasonic probe 110 and the insertion tube 130. In particular, the bending part 120 is configured to enable the ultrasonic probe 110 to be easily inserted through a curved esophagus and positioned at a desired diagnosis position.

The bending part 120 may include a plurality of segments and manipulation wires which are configured to connect the respective segments to each other. Such a bending part 120 composed of the segments is formed in a hollow cylindrical shape, in which a signal transmission cable which is configured to transmit a signal from a back-end system to the ultrasonic probe 110 and vice versa is accommodated.

The bending part 120 may be made of a metal, however, the exemplary embodiments are not limited thereto. Any material having a relatively high thermal conductivity and certain intensity may be used for the bending part 120.

An outer wall of the bending part 120 may be coated with a material having a relatively high thermal conductivity. Thereby, heat transferred to the inner wall of the bending part 120 via the graphite 210 may be easily dissipated to the outside via the outer wall of the bending part 120. Accordingly, heat generated by the integrated circuit 112 is dissipated to the outside via the heat spreader 114, the graphite 210 and the bending part 120.

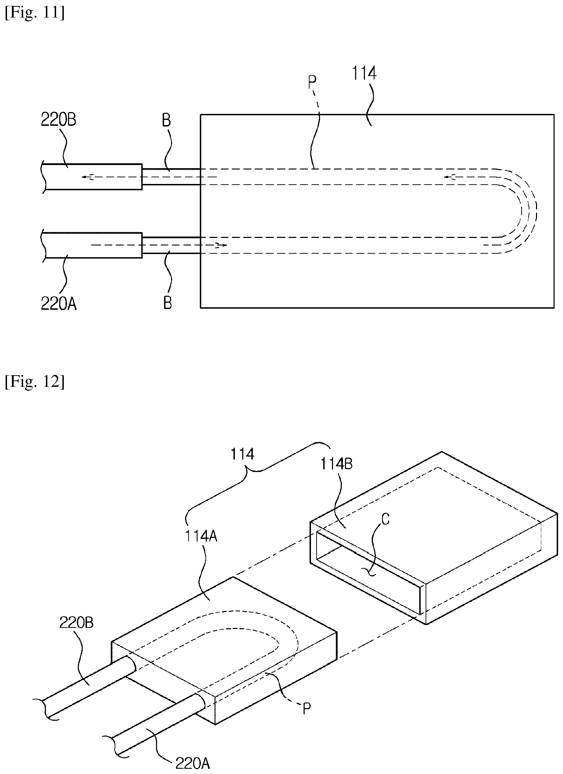

FIGS. 10 and 11 are views illustrating a case in which a refrigerant circulation device is used as the heat transferer.

In this exemplary embodiment, cooling water is used as the refrigerant, however, the refrigerant is not limited to cooling water. The refrigerant circulation device may include a refrigerant circulation tube 220 configured to circulate the refrigerant therethrough and a water pump 221 configured to supply the refrigerant to the heat spreader 114 and to receive the refrigerant passing through the heat spreader 114, however the refrigerant circulation device is not limited to the above structure.

Referring to FIGS. 10 and 11, the protruding parts B of the heat spreader 114 and the refrigerant circulation tube 220 are connected to each other. In particular, one of the pair of protruding parts B may be connected to a first refrigerant circulation tube 220A, and the other of the pair of protruding parts B may be connected to a second refrigerant circulation tube 220B.

The refrigerant circulation tube 220 may have one end connected to the protruding part B of the heat spreader 114 and an opposite end connected to the water pump 221. Accordingly, as shown in FIG. 11, the refrigerant flowing into the first refrigerant circulation tube 220A from the water pump 221 flows into a passage P formed in the heat spreader 114 through the protruding part B. The refrigerant passing through the passage P flows into the second refrigerant circulation tube 220B through the protruding part B. The refrigerant passing through the second refrigerant circulation tube 220B flows back into the water pump 221.

Because the refrigerant flowing back into the water pump 221 after circulating within the interior of the heat spreader 114 carries the heat absorbed in the heat spreader 114 from the integrated circuit 112 to the water pump 221, the temperature of the refrigerant in the water pump 221 increases. Accordingly, as shown in FIG. 10, the refrigerant circulation device may further include a heat sink 223 arranged adjacent to the water pump 221.

The heat sink 223 is configured to disperse the heat carried to the water pump 221 in order to dissipate the heat to the outside. Although not illustrated in FIG. 10, the heat sink 223 may include a heat radiation plate and a heat radiation fan, however, the heat sink is not limited to the above structure. The heat sink 223 may include only the heat radiation plate, or may include only the heat radiation fan. The heat radiation plate may include a plurality of fins which are made of a metal having a relatively high thermal conductivity, such as, for example, aluminum, however, the exemplary embodiments are not limited thereto.

As described above, heat absorbed in the heat spreader 114 is carried to the water pump 221 by circulating the refrigerant through an internal passage 114c of the heat spreader 114 which has absorbed heat from the integrated circuit 112, and then the heat carried to the water pump 221 is dissipated to the outside by using the heat sink 223.

The refrigerant circulation tube 220 may be directly connected to the protruding part B of the heat spreader 114 as described above. Alternatively, as shown in FIG. 12, the heat spreader 114 may be structured to include an inner casing 114A which is connected to the refrigerant circulation tube 220 and an outer casing 114B which is formed with a coupling recess C having a shape which corresponds to the inner casing 114A.

This is because it may be difficult to directly connect the refrigerant circulation tube 220 to the relatively small-sized protruding part B of the heat spreader 114. In this case, a passage P through which the refrigerant circulates may be formed inside the inner casing 114A.

The heat spreader 114 according to an exemplary embodiment, as shown in FIG. 12, may be realized by inserting the inner casing 114A, which is coupled to the refrigerant circulation tube 220 and has the passage P formed therein to be connected to the refrigerant circulation tube 220 and to facilitate refrigerant circulation, into the coupling recess C formed at the outer casing 114B. The coupling between the inner casing 114A and the outer casing 114B may be achieved by a common cartridge coupling, however, the exemplary embodiments are not limited thereto. The coupling between the inner casing 114A and the outer casing 114B may be achieved in any well-known coupling manner.

The insertion tube 130, as shown in FIG. 1, may have one end connected to the bending part 120 and an opposite end connected to the manipulation device 140. The insertion tube 130 may be flexible enough to smoothly pass through an esophagus and durable enough to resist tearing easily. In general, the insertion tube 130 may have a length ranging from about 100 cm to about 110 cm and a diameter ranging from about 10 mm to about 20 mm, however, the exemplary embodiments are not limited thereto.

The manipulation device 140 functions to enable a user to manipulate the operation of the ultrasonic probe 110. For example, as shown in FIG. 1, the manipulation device 140 may include a first knob 141 which is configured to move the ultrasonic probe 110 from side to side and a second knob 142 which is configured to move the ultrasonic probe 110 up and down, however, the exemplary embodiments are not limited thereto.

The movement of the ultrasonic probe 110 from side to side and up and down by the manipulation device 140 will presently be described.

Although not illustrated in FIG. 1, the bending part 120 having a multi-joint mechanism may include a plurality of segments and manipulation wires which are configured to connect the respective segments to each other. By moving the respective segments using the manipulation wires, the bending part 120 may be bent from side to side and up and down.

When a user rotates the first knob 141 in one direction or in the reverse direction, the corresponding manipulation wire is manipulated (for example, is pulled), so that the bending part 120 is bent left or right. As a result, the ultrasonic probe 110, which is affixed to a distal end portion of the bending part 120, is also moved left or right. When a user rotates the second knob 142 in one direction or in the reverse direction, the corresponding manipulation wire is manipulated, so that the bending part 120 is bent up or down. As a result, the ultrasonic probe 110, which is affixed to the distal end portion of the bending part 120, is also moved up or down.

Although the mechanism in which the ultrasonic probe 110 is operated by manipulating the manipulation device 140 has been described, this is merely illustrative. The operation mechanism of the ultrasonic probe 110 is not limited to the above-described mechanism.

Although not illustrated in FIG. 1, the ultrasonic imaging apparatus 100 according to an exemplary embodiment may further include a back-end system (not shown) which is configured to transmit electrical signals to the ultrasonic probe 110, to generate an ultrasonic image by using the electrical signals received from the ultrasonic probe 110, and to display the generated ultrasonic image.

The ultrasonic imaging apparatus 100 according to an exemplary embodiment may further include a connector 160 connected to the back-end system (not shown) and a cable 150 which is configured to connect the connector 160 and the manipulation device 140 to each other.

## Claims

1. An ultrasonic imaging apparatus (100) comprising:
an ultrasonic probe (110) arranged at a distal end portion of the ultrasonic imaging apparatus (100), and
a bending part (120) connected to the ultrasonic probe (110) and configured to be bendable,
wherein heat generated by the ultrasonic probe (100) is dissipated toward the bending part (120),
wherein the ultrasonic probe (110) includes:
an ultrasonic transducer (111);
an integrated circuit (112) which includes a front surface and a rear surface, the ultrasonic transducer (111) being arranged on the front surface of the integrated circuit (112);
a printed circuit board (113) which includes a front surface and a rear surface, the rear surface of the integrated circuit (112) being arranged on the front surface of the printed circuit board (113); and
a heat spreader (114) configured to absorb heat generated by the integrated circuit (112), the beat spreader (114) being arranged on the rear surface of the printed circuit board (113);
the ultrasound imaging apparatus further comprising:
a heat transferer connected to the heat spreader (114) and configured to transfer heat absorbed by the heat spreader (114) toward the bending part (120),
wherein the heat transferer includes a refrigerant circulation device,
wherein the refrigerant circulation device includes a refrigerant circulation tube (220) configured to circulate a refrigerant therethrough,
wherein the heat spreader (114) includes:
an inner casing (114A) coupled to the refrigerant circulation tube (220) and having a passage (P) formed therein configured to circulate the refrigerant therethrough; and
an outer casing (114B) formed with a coupling recess (C) and having a shape which corresponds to the inner casing (114A).

2. The ultrasonic imaging apparatus (100) according to claim 1, wherein the heat spreader (114) further includes a protruding part (A) having a pipe shape, the protruding part (A) being connected to the refrigerant circulation tube (220).

3. The ultrasonic imaging apparatus (100) according to claim 2, wherein the heat spreader (114) further includes a passage formed therein configured to circulate the refrigerant therethrough.

4. The ultrasonic imaging apparatus (100) according to claim 1, wherein the refrigerant includes cooling water, and the refrigerant circulation device includes a water pump (221) configured to supply the cooling water to the heat spreader (114) and to receive the cooling water passing through the heat spreader (114), wherein the refrigerant circulation device further includes a heat sink (223) arranged adjacent to the water pump (221) and configured to dissipate heat transferred from the heat spreader (114) to an outside.

5. The ultrasonic imaging apparatus (100) according to claim 1, wherein the heat transferer includes graphite (210).

6. The ultrasonic imaging apparatus (100) according to claim 5, wherein the heat spreader (114) further includes a protruding part (B) having a plate shape, wherein the graphite (210) includes a first end which is bonded to the protruding part (B) and a second end which is bonded to an inner wall of the bending part (120).

7. The ultrasonic imaging apparatus (100) according to claim 1, wherein the ultrasonic transducer (111) includes a capacitive micromachined ultrasonic transducer.

8. The ultrasonic imaging apparatus (100) according to claim 1, wherein the printed circuit board (113) is formed with an opening (113a) which is configured to expose the rear surface of the integrated circuit (112) therethrough.

9. The ultrasonic imaging apparatus (100) according to claim 8, wherein the printed circuit board (113) further includes at least one from among a thermal grease and a phase change material, the at least one from among the thermal grease and the phase change material being provided in the opening.

10. The ultrasonic imaging apparatus (100) according to claim 1, wherein the printed circuit board (113) includes a plurality of vias (113b) provided through the printed circuit board (113) in a thickness direction of the printed circuit board (113), wherein each of the plurality of vias (113b) is made of copper.

## Patentansprüche

1. Eine Ultraschallabbildungsvorrichtung (100), aufweisend:
eine Ultraschallsonde (110), die an einem distalen Endabschnitt der Ultraschallabbildungsvorrichtung (100) angeordnet ist, und
einen Biegeteil (120), der mit der Ultraschallsonde (110) verbunden ist und konfiguriert ist, um biegbar zu sein,
wobei Wärme, die von der Ultraschallsonde (100) erzeugt wird, zum Biegeteil (120) hin abgeleitet wird,
wobei die Ultraschallsonde (110) aufweist:
einen Ultraschallwandler (111);
eine integrierte Schaltung (112), die eine Vorderfläche und eine Rückfläche aufweist, wobei der Ultraschallwandler (111) auf der Vorderfläche der integrierten Schaltung (112) angeordnet ist;
eine Leiterplatte (113), die eine Vorderfläche und eine Rückfläche aufweist, wobei die Rückfläche der integrierten Schaltung (112) auf der Vorderfläche der Leiterplatte (113) angeordnet ist; und
eine Wärmeverteilungsvorrichtung (114), die konfiguriert ist, um Wärme, die von der integrierten Schaltung (112) erzeugt wird, zu absorbieren, wobei die Wärmeverteilungsvorrichtung (114) auf der Rückfläche der Leiterplatte (113) angeordnet ist,
wobei die Ultraschallabbildungsvorrichtung ferner aufweist:
eine Wärmeübertragungsvorrichtung, die mit der Wärmeverteilungsvorrichtung (114) verbunden ist und konfiguriert ist, um Wärme, die von der Wärmeverteilungsvorrichtung (114) absorbiert wird, zum Biegeteil (120) hin zu übertragen,
wobei die Wärmeübertragungsvorrichtung eine Kühlmittelzirkulationsvorrichtung aufweist,
wobei die Kühlmittelzirkulationsvorrichtung ein Kühlmittelzirkulationsrohr (220), das konfiguriert ist, um ein Kühlmittel hindurch zirkulieren zu lassen, aufweist,
wobei die Wärmeverteilungsvorrichtung (114) aufweist:
ein inneres Gehäuse (114A), das mit dem Kühlmittelzirkulationsrohr (220) gekoppelt ist und einen Durchgang (P), der im inneren Gehäuse ausgebildet ist und konfiguriert ist, um das Kühlmittel durch ihn hindurch zirkulieren zu lassen, aufweist; und
ein äußeres Gehäuse (114B), das mit einer Kopplungsausnehmung (C) ausgebildet ist und eine Form, die dem inneren Gehäuse (114A) entspricht, aufweist.

2. Die Ultraschallabbildungsvorrichtung (100) nach Anspruch 1, wobei die Wärmeverteilungsvorrichtung (114) ferner einen vorstehenden Teil (A), der eine Rohrform aufweist, aufweist, wobei der vorstehende Teil (A) mit dem Kältezirkulationsrohr (220) verbunden ist.

3. Die Ultraschallabbildungsvorrichtung (100) nach Anspruch 2, wobei die Wärmeverteilungsvorrichtung (114) ferner einen Durchgang, der darin ausgebildet ist und konfiguriert ist, um das Kühlmittel durch ihn hindurch zirkulieren zu lassen, aufweist.

4. Die Ultraschallabbildungsvorrichtung (100) nach Anspruch 1, wobei das Kühlmittel Kühlwasser aufweist und die Kühlmittelzirkulationsvorrichtung eine Wasserpumpe (221), die konfiguriert ist, um das Kühlwasser der Wärmeverteilungsvorrichtung (114) zuzuführen und das Kühlwasser, das durch die Wärmeverteilungsvorrichtung (114) strömt, zu erhalten, aufweist, wobei die Kühlmittelzirkulationsvorrichtung ferner einen Kühlkörper (223), der benachbart zur Wasserpumpe (221) angeordnet ist und konfiguriert ist, um Wärme, die von der Wärmeverteilungsvorrichtung (114) übertragen wird, nach außen abzuleiten.

5. Die Ultraschallabbildungsvorrichtung (100) nach Anspruch 1, wobei die Wärmeübertragungsvorrichtung (210) Graphit aufweist.

6. Die Ultraschallabbildungsvorrichtung (100) nach Anspruch 5, wobei die Wärmeverteilungsvorrichtung (114) ferner einen vorstehenden Teil (B), der eine Plattenform aufweist, aufweist, wobei das Graphit (210) ein erstes Ende, das mit dem vorstehenden Teil (B) verbunden ist, und ein zweites Ende, das mit einer Innenwand des Biegeteils (120) verbunden ist, aufweist.

7. Die Ultraschallabbildungsvorrichtung (100) nach Anspruch 1, wobei der Ultraschallwandler (111) einen kapazitiven mikrobearbeiteten Ultraschallwandler aufweist.

8. Die Ultraschallabbildungsvorrichtung (100) nach Anspruch 1, wobei die Leiterplatte (113) mit einer Öffnung (113a), die konfiguriert ist, um die Rückfläche der integrierten Schaltung (112) durch sie hindurch freizulegen, ausgebildet ist.

9. Die Ultraschallabbildungsvorrichtung (100) nach Anspruch 8, wobei die Leiterplatte (113) ferner zumindest eines aus einer Wärmeleitpaste und einem Phasenwechselmaterial aufweist, wobei das zumindest eine aus der Wärmeleitpaste und dem Phasenwechselmaterial in der Öffnung bereitgestellt wird.

10. Die Ultraschallabbildungsvorrichtung (100) nach Anspruch 1, wobei die Leiterplatte (113) eine Vielzahl von Durchkontaktierungen (113b), die durch die Leiterplatte (113) hindurch in einer Dickenrichtung der Leiterplatte (113) bereitgestellt werden, aufweist, wobei jede der Vielzahl der Durchkontaktierungen (113b) aus Kupfer besteht.

## Revendications

1. Un appareil d'imagerie ultrasonore (100), comprenant:
une sonde ultrasonore (110) disposée au niveau d'une partie d'extrémité distale de l'appareil d'imagerie ultrasonore (100), et
une partie de pliage (120) reliée à la sonde ultrasonore (110) et configurée pour être pliable,
de la chaleur générée par la sonde ultrasonore (100) étant dissipée vers la partie de pliage (120),
la sonde ultrasonore (110) comprenant :
un transducteur ultrasonore (111) ;
un circuit intégré (112) qui comprend une surface avant et une surface arrière, le transducteur ultrasonore (111) étant disposé sur la surface avant du circuit intégré (112) ;
une carte de circuit imprimé (113) qui comprend une surface avant et une surface arrière, la surface arrière du circuit intégré (112) étant disposée sur la surface avant de la carte de circuit imprimé (113) ; et
un diffuseur de chaleur (114) configuré pour absorber de la chaleur générée par le circuit intégré (112), le diffuseur de chaleur (114) étant disposé sur la surface arrière de la carte de circuit imprimé (113),
l'appareil d'imagerie ultrasonore comprenant en outre :
un appareil de transfert de chaleur relié au diffuseur de chaleur (114) et configuré pour transférer de la chaleur absorbée par le diffuseur de chaleur (114) vers la partie de pliage (120),
l' appareil de transfert de chaleur comprenant un dispositif de circulation de réfrigérant,
le dispositif de circulation de réfrigérant comprenant un tube de circulation de réfrigérant (220) configuré pour faire circuler un réfrigérant par celui-ci, le diffuseur de chaleur (114) comprenant :
un boîtier intérieur (114A) couplé au tube de circulation de réfrigérant (220) et ayant un passage (P) formé dans le boîtier intérieur et configuré pour faire circuler le réfrigérant par le passage ; et
un boîtier extérieur (114B) formé avec un évidement de couplage (C) et ayant une forme qui correspond au boîtier intérieur (114A).

2. L'appareil d'imagerie ultrasonore (100) selon la revendication 1, dans lequel le diffuseur de chaleur (114) comprend en outre une partie saillante (A) présentant une forme de tuyau, la partie saillante (A) étant reliée au tube de circulation de réfrigérant (220).

3. L'appareil d'imagerie ultrasonore (100) selon la revendication 2, dans lequel le diffuseur de chaleur (114) comprend en outre un passage formé dans celui-ci et configuré pour faire circuler le réfrigérant par le passage.

4. L'appareil d'imagerie ultrasonore (100) selon la revendication 1, dans lequel le réfrigérant comprend de l'eau de refroidissement, et le dispositif de circulation de réfrigérant comprend une pompe à eau (221) configurée pour fournir l'eau de refroidissement au diffuseur de chaleur (114) et pour recevoir l'eau de refroidissement traversant le diffuseur de chaleur (114), le dispositif de circulation de réfrigérant comprenant en outre un dissipateur thermique (223) disposé adjacent à la pompe à eau (221) et configuré pour dissiper de la chaleur transférée par le diffuseur de chaleur (114) vers l'extérieur.

5. L'appareil d'imagerie ultrasonore (100) selon la revendication 1, dans lequel le dispositif de transfert de chaleur comprend du graphite (210).

6. L'appareil d'imagerie ultrasonore (100) selon la revendication 5, dans lequel le diffuseur de chaleur (114) comprend en outre une partie saillante (B) présentant une forme de plaque, le graphite (210) comprenant une première extrémité qui est collée à la partie saillante (B) et une seconde extrémité qui est collée à une paroi intérieure de la partie de pliage (120).

7. L'appareil d'imagerie ultrasonore (100) selon la revendication 1, dans lequel le transducteur ultrasonore (111) comprend un transducteur ultrasonore micro-usiné capacitif.

8. L'appareil d'imagerie ultrasonore (100) selon la revendication 1, dans lequel la carte de circuit imprimé (113) est formée avec une ouverture (113a) qui est configurée pour exposer la surface arrière du circuit intégré (112) par celle-ci.

9. L'appareil d'imagerie ultrasonore (100) selon la revendication 8, dans lequel la carte de circuit imprimé (113) comprend en outre une graisse thermique et/ou un matériau à changement de phase, la graisse thermique et/ou le matériau à changement de phase étant prévu/e/s dans l'ouverture.

10. L'appareil d'imagerie ultrasonore (100) selon la revendication 1, dans lequel la carte de circuit imprimé (113) comprend une pluralité de trous d'interconnexion (113b) prévus de manière à traverser la carte de circuit imprimé (113) dans une direction d'épaisseur de la carte de circuit imprimé (113), chacun de la pluralité de trous d'interconnexion (113b) étant fait en cuivre.
